# EUROPÄISCHE PATENTSCHRIFT

(11) **EP 2 970 562 B1**
(45) Veröffentlichungstag und Bekanntmachung des Hinweises auf die Patenterteilung: **08.08.2018**
(21) Anmeldenummer: 14708543.5
(22) Anmeldetag: 07.03.2014
(51) Int. Cl.: C08G 18/79, C08G 18/02, C08G 18/18

(54) **VERFAHREN ZUR HERSTELLUNG VON TDI-TRIMERISATEN MIT BESONDERER REINHEIT**
METHOD FOR THE PREPARATION OF ESPECIALLY PURE TDI TRIMERISATES
PROCÉDÉ DE FABRICATION DE TRIMÈRES DE TDI D'UNE GRANDE PURETÉ

(30) Priorität: 12.03.2013 EP 13158688
(43) Veröffentlichungstag der Anmeldung: 20.01.2016
(73) Patentinhaber: Covestro Deutschland AG, 51373 Leverkusen (DE)
(72) Erfinder: SANDERS, Josef, 51375 Leverkusen (DE); HECKING, Andreas, 40764 Langenfeld (DE); HALPAAP, Reinhard, 51519 Odenthal (DE); RICHTER, Frank, 51373 Leverkusen (DE); WILMES, Oswald, 51061 Köln (DE); BUSCH, Jan, 40477 Düsseldorf (DE); LODDENKEMPER, Tim, 41542 Dormagen (DE); STEFFENS, Friedhelm, 51373 Leverkusen (DE); GROTH, Stefan, 51375 Leverkusen (DE)
(74) Vertreter: Levpat
(86) Internationale Anmeldenummer: PCT/EP2014/054434
(87) Internationale Veröffentlichungsnummer: WO 2014/139879

(56) Entgegenhaltungen:
- EP-A1- 1 378 529
- EP-A1- 1 378 530
- WO-A1-2005/070984
- DE-A1- 4 428 107
- HACH Lange GmbH: "Objective Color Assessment and Quality Control in the Chemical, Pharmaceutical and Cosmetic Industries", 2016

## Beschreibung

Die Erfindung betrifft ein neues Verfahren zur Herstellung von isocyanatgruppenhaltigen Polyisocyanuraten auf Basis von 2,4- und 2,6-Toluylendiisocyanat (TDI) und deren Verwendung in Beschichtungsmitteln.
Die Herstellung von isocyanatgruppenhaltigen Polyisocyanuraten ist seit langem bekannt und in einer Vielzahl von Veröffentlichungen und Patenten beschrieben (Houben-Weyl, Methoden der organischen Chemie Band 8, S. 136f, Georg Thieme Verlag Stuttgart 1952, H Wagner, H F. Sarx, Lackkunstharze 5 Auflage, Seite 153ff, Carl Hanser Verlag München 1971; DE-A 4 428 107, US-PS 2 993 870; DE-C 1 201 992, DE-A 2 452 532, J prakt. Chem 336, S 185 bis 200, 1994). Sowohl Trimerisate auf Basis aliphatischer als auch aromatischer Diisocyanate werden universell als Lackrohstoffe sowie als Polyurethanelastomere und Polyurethanschäume eingesetzt
Handelsübliche Produkte werden heutzutage so hergestellt, dass man großtechnisch verfugbare Gemische aus 2,4-TDI und 2,6-TDI unter Katalyse durch Dialkylaminogruppen enthaltende phenolische Katalysatoren (Mannichbasen) in geeigneten organischen Losungsmitteln bis zum nahezu vollstandigen Umsatz trimerisiert und den Katalysator anschließend durch Zusatz von sauer reagierenden Substanzen oder durch Umsetzung mit Alkylierungsmittel deaktiviert Diese Deaktivierung ist erforderlich, da die Produkte ansonsten nicht stabil sind, was sich durch einen Abfall des NCO-Gehalts und einen Anstieg der Viskostat mit der Zeit bemerkbar macht.
Aus arbeitshygienischen Grunden werden als Produkte heute monomerenarme Trimerisat-Typen bevorzugt Hergestellt werden diese Produkte entweder durch destillatives Abtrennen des überschüssigen Monomers nach erfolgter Trimerisationsreaktion oder durch Steuerung der Trimerisationsreaktion zu entsprechend hohen Umsätzen, bis das Monomer sich weitestgehend zu hoheroligomeren Isocyanuraten umgesetzt hat. Letzteres Verfahren führt besonders dann zum Erfolg, wenn die eingesetzten Diisocyanate wie im Falle von 2,4-Toluylendiisocyanat - zwei deutlich unterschiedlich reaktive Isocyanatgruppen tragen Entsprechende Losemittel enthaltende Produkte konnen so mit einem Gehalt an monomerem TDI (Summe der isomeren Toluylendiisocyanate) von < 0,5 % hergestellt werden (z.B Desmodur® IL,Verkaufsprodukt der Bayer AG, 50 %-ig in Butylacetat, NCO-Gehalt 8,0 %) Die EP 1 378 529 A1 beschreibt ein Verfahren zur Herstellung monomerenarmer Isocyanuratgruppen enthaltender Polyisocyanate auf Basis 2,4- und 2,6-TDI ohne physikalische Abtrennung von monomerem TDI.
Die auf diese Weise hergestellten Polyisocyanate des Standes der Technik weisen jedoch den grundlegenden Nachteil auf, dass sie herstellungsbedingt immer durch die abgestoppten Katalysatoren verunreinigt sind, wobei deren Gehalt stark von der Reaktivitat des eingesetzten Toluylendiisocyanat bzw. von der jeweils benotigten Katalysator-Menge abhangig ist Diese Verunreinigungen durch die abgestoppten Katalysatoren fuhren zu einer schnelleren Vergilbung und Alterung der Polyisocyanate und der damit hergestellten Lacke
Ursache für den erhöhten Katalysatorverbrauch und die dadurch verursachte Verfärbung der Polyisocyanate sowie der damit hergestellten Lacke ist insbesondere die Verwendung von TDI mit nicht ausreichender Reinheit. Es hat daher nicht an Versuchen gefehlt, TDI-Qualitäten bereitzustellen, mit denen farbhellere und alterungsstabilere aromatische Polyisocyanate hergestellt werden können. Beispielsweise wird in der EP 1 413 571 ein Verfahren beschrieben, mit dem durch Vorkonzentrierung der TDI-Rohlösung auf einen Lösungsmittelgehalt von <20% und anschließende Fraktionierung in einer Trennwanddestillationskolonne eine Produktfraktion mit einem TDI-Gehalt von mindestens 99,5% und weniger als 200 Gew.-ppm Lösungsmittel und/oder chlorierte aromatische Kohlenwasserstoffe, weniger als 100 Gew.-ppm hydrolisierbares Chlor und weniger als 40 Gew.-ppm Säure erhalten wird. In US-PS 6,900,348 bzw. in der entsprechenden EP 1 187 808 wird beschrieben, dass durch Verwendung von Phosgen mit einem Brom-Gehalt von <50 ppm farbhellere Diphenylmethan-diisocyanate erhalten werden können. Die EP 0 816 333 beansprucht ein Verfahren zur Verringerung der Farbe von TDI durch Behandlung der Rohlösung mit Wasserstoff vor der Abtrennung des Lösungsmittels.

Auch eine spezielle Vorbehandlung des zur Herstellung von TDI verwendeten Toluylendiamins (TDA) kann zu einer verbesserten Reinheit des TDIs führen. Beispielsweise beansprucht die EP 1 864 969 ein Verfahren zur Herstellung von farbhellerem TDI, bei dem das hierfür in die Phosgenierung eingesetzte TDA weniger als 0,1 Gew.-% alkylierte cyclische Ketone bezogen auf 100 Gew.-% TDA enthält. In der US-PS 5,872,278 bzw. der entsprechenden EP 0 866 057 wird ein Verfahren beschrieben, bei dem das eingesetzte Amin vor der Umsetzung mit Phosgen mit Feststoffen behandelt wird, die Lewis- und/oder Brönstedt-Säure-Zentren enthalten. Die erhaltenen Isocyanate weisen dann eine hellere Farbe auf, als Isocyanate, die mit unbehandeltem Amin hergestellt wurden.

Mit diesen vergleichsweise sehr aufwändigen Verfahren können zwar TDI-Qualitäten mit größerer Reinheit und hellerer Farbe hergestellt werden, jedoch findet sich dort kein Hinweis darauf, welche Nebenkomponenten für den erhöhten Katalysatorverbrauch und die noch nicht ausreichend zu verhindernde Verfärbung der Polyisocyanate und der damit hergestellten Lacke verantwortlich sind und wie diese Verfärbung in ausreichendem Maß verhindert werden kann. Es besteht daher immer noch ein dringender Bedarf an farbhellen alterungsstabilen aromatischen Lackpolyisocyanaten.

Aufgabe der vorliegenden Erfindung war es daher, ein Verfahren zu finden, mit dem der Gehalt an abgestoppten Katalysatoren in den Polyisocyanten soweit reduziert werden kann, dass damit farbhellere und alterungsbeständigere Polyisocyanate und Lacke hergestellt werden können.

Diese Aufgabe konnte mit dem nachstehend näher beschriebenen Verfahren gelöst werden.

Der Erfindung liegt die überraschende Beobachtung zugrunde, dass der Katalysator-Verbrauch bei der Herstellung der isocyanatgruppenhaltigen Polyisocyanurate auf Basis von Toluylendiisocyanat deutlich reduziert werden kann, wenn man zu ihrer Herstellung Toluylendiisocyanat einsetzt, das einen Gehalt an 2-Chloro-6-isocyanato-methylcyclohexadienen (CIMCH) von <5 Gew.-ppm aufweist. Mit den nach diesem Verfahren hergestellten farbhellen Polyisocyanuraten können farbhellere und alterungsbeständigere Lacke hergestellt werden. Farbhell in diesem Zusammenhang bedeutet, dass die solcherart hergestellten Polyisocyanate APHA-Farbzahlen von <100 Hazen bevorzugt <75 Hazen, besonders bevorzugt <55 Hazen, gemessen auf Basis der DIN EN 1557, aufweisen.

CIMCH kann in Form von 3 Doppelbindungsisomeren vorliegen, die im TDI in unterschiedlichem Verhältnis enthalten sein können. Diese werden z. B. bei der TDI-Herstellung aus im eingesetzten TDA enthaltenen 1-Amino-2-methyl-cyclohexenon gebildet, das wiederum bei der Herstellung von TDA aus Dinitrotoluol (DNT) durch partielle Kernhydrierung von TDA und Ersatz einer Aminofunktion durch Wasser entstehen kann. Es ist auch möglich, dass die Ketofunktion bereits anteilig durch oxidativen Angriff bei der Herstellung von DNT durch Nitrierung von Toluol eingeführt wird, wobei zunächst Nitrokresole entstehen, die dann bei der anschließenden Hydrierung das oben beschriebene 1-Amino-methyl-2-cyclohexenon bilden können.

Gegenstand der Erfindung ist somit ein Verfahren zur Herstellung Lösemittel und/oder Verdünnungsmittel, sowie Isocyanuratgruppen enthaltender Polyisocyanate auf Basis 2,4- und/oder 2,6- Toluylendiisocyanat mit einem Gehalt an monomerem Diisocyanat von < 0,5 Gew.-% bezogen auf Polyisocyanat plus Lösemittel, durch Trimerisierung von
A) 20 bis 80 Gew.-% großtechnisch verfügbarer Gemische bestehend im Wesentlichen aus 2,4- Toluylendiisocyanat und 2,6- Toluylendiisocyanat enthaltend 65 bis 95 Gew.-% 2,4-Toluylendiisocyanat und 5 bis 35 Gew.-% 2,6- Toluylendiisocyanat in Gegenwart von
B) 20 bis 80 Gew.-% Lösemitteln und/oder Verdünnungsmitteln sowie
C) Dialkylaminomethylgruppen enthaltenden phenolischen Katalysatoren
bei einer Temperatur von 40 bis 120°C bis zum nahezu vollständigen Umsatz und anschließende Deaktivierung des Katalysators durch Zusatz von sauer reagierenden Substanzen oder durch Umsetzung mit Alkylierungsmitteln, dadurch gekennzeichnet,
dass das eingesetzte Toluylendiisocyanat einen Gehalt an 2-Chloro-6-isocyanato-methylcyclohexadienen (CIMCH) von <5 Gew.-ppm aufweist.

Ein nahezu vollständiger Umsatz bedeutet, dass das eingesetzte TDI bis auf einen Restmonomerengehalt von < 0,5 Gew.-% bezogen auf Polyisocyanat plus Lösemittel umgesetzt wird.

Gegenstand der Erfindung sind auch die nach diesem Verfahren hergestellten Lösemittel und/oder Verdünnungsmittel sowie Isocyanuratgruppen enthaltenden Polyisocyanate, sowie deren Verwendung als Polyisocyanatkomponente in Polyurethanlacken, insbesondere in Zweikomponenten-Polyurethanlacken.

Als Toluylendiisocyanat A) kommen insbesondere 2,4-Toluylendiisocyanat und seine technischen Gemische mit bis zu 35 Gew.-%, bezogen auf Gemisch, an 2,6-Toluylendiisocyanat in Betracht, die einen Gehalt an 2-Chloro-6-isocyanatomethylcyclohexadienen (CIMCH) von <5 Gew.-ppm aufweisen. Derartige TDI-Qualitäten können beispielsweise durch gezielte destillative Entfernung von 2-Chloro-6-isocyanatomethylcyclohexadienen aus den vorkonzentrierten TDI-Rohlösungen in einer Trennwanddestillationskolonne erhalten werden, wie dies in der EP 1 413 571 B1 beschrieben ist. Besonders bevorzugt sind jedoch Toluylendiisocyanate, die durch Gasphasenphosgenierung von TDA hergestellt werden und deren Gehalt an 2-Chloro-6-isocyanato-methylcyclohexadienen unterhalb der Nachweisgrenze liegt. Toluylendiisocyanat einer derartigen Qualität ist z.B. von der Bayer Material Science AG aus der Produktion am Standort Caojing in China erhältlich.

Zur eindeutigen Charakterisierung der Komponente 2-Chloro-6-isocyanatomethylcyclohexadienen wurden zwei voneinander unabhängige analytische Methoden genutzt. Mittels gaschromatographischer Techniken wurden unterschiedliche Toluylendiisocyanat-Qualitäten mit einem 2,4-Anteil von rund 80 Gew.-% auf ihre Ungleichheiten in dem Nebenkomponentenspektrum hin untersucht. Durch anschließende gekoppelte gaschromatographische Massenspektroskopie wurde den bis dahin drei unbekannten Verbindungen (CIMICH einschließlich zweier Isomeren) ein Molekulargewicht von 169g/mol zugeordnet. Weitere Strukturinformationen konnten durch dem Fachmann bekannte Weise aus der Fragmentierung gewonnen werden. Mittels aufwendigen kernresonanzspektroskopischen Experimenten (¹H-NMR, ¹H-COSY, ¹H-,¹H-TOCSY und ¹H-,¹³C-HMBC) konnten den drei Komponenten mit m/z 169 die unten aufgeführten Strukturen zu geordnet werden.

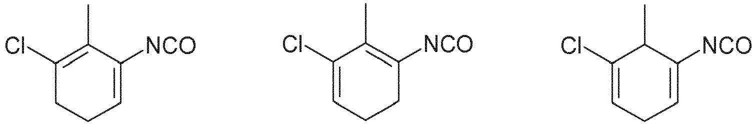

Durch gezielte Methodenentwicklung konnte die Nachweisgrenze der Isomeren des CIMICH mittels gaschromatographischer Spektroskopie, unter Verwendung einer Optima 5 HT Säule (60m Länge, 0,25mm Innendurchmesser, 0,25µm Filmdicke) von Macherey-Nagel in einem HP Series 6890 Gaschromatographen von Hewlett Packard auf 1 Gew.-ppm festgelegt werden.

Als Lösemittel B) können in der Polyurethanchemie gebräuchliche Verdünnungsmittel und Lösemittel wie beispielsweise Toluol, Xylol, Cyclohexan, Chlorbenzol, Butylacetat, Ethylacetat, Ethylglykolacetat, Pentylacetat, Hexylacetat, Methoxypropylacetat, Tetrahydrofuran, Dioxan, Aceton, N-Methylpyrrolidon, Methylethylketon, Testbenzin, höhersubstituierte Aromaten, wie beispielsweise unter der Bezeichnung Solvent Naphtha®, Solvesso®, Shellsol®, Isopar®, Nappar® und Diasol® im Handel, Schwerbenzol, Tetralin, Dekalin und Alkane mit mehr als 6 Kohlenstoffatomen, üblicheWeichmacher, wie Phthalate, Benzoate, Sulfonsäureester und Phosphorsäureester sowie Gemische derartiger Verdünnungs- und Lösungsmittel eingesetzt werden. Die Konzentration des Verdünnungs- und Lösemittels wird hierbei auf 20 bis 80 Gew.-%, vorzugsweise 40 bis 60 Gew.- % eingestellt.

Ferner geeignet als Lösemittel B) sind auch Polyisocyanate auf Basis aliphatischer Diisocyanate wie sie z.B.in der DE-A 4 428 107 beschrieben sind. Hiermit sind verdünnte monomerenarme TDI-Trimerisate zugänglich, die keine leicht verdampfbaren Lösemittel und Verdünnungsmittel enthalten.

Als phenolische Katalysatoren C) zur Initiierung und Beschleunigung der Trimerisationsreaktion kommen als Mannichbasen spezielle Systeme mit sogenanntem negativem Temperatureffekt in Betracht, die auch bei höheren Temperaturen zu einem selektiven Einbau von TDI führen. Solche Katalysatorsysteme weisen an Aromaten gebundene N,N-Dialkylaminomethylgruppen und phenolische OH-Gruppen auf. Bei den Alkylgruppen handelt es sich um unterschiedliche oder gleiche Reste mit je bis zu 18 Kohlenstoffatomen, die gegebenenfalls durch Sauerstoff oder Schwefel getrennt sind, oder um verbrückende Alkylgruppen, in Form einer gegebenenfalls Sauerstoff oder Schwefel enthaltende Alkylengruppe mit bis zu 18 Kohlenstoffatomen. Die N,N-Dialkylaminomethylgruppen und die phenolischen OH-Gruppen können auf mehrere Moleküle verteilt, oder an einem oder mehreren benzolischen Aromaten positioniert sein. Vorzugweise werden Verbindungen als Katalysatorsysteme eingesetzt, die sowohl Hydroxyl- als auch Dialkylaminomethlyguppen in einem Molekül enthalten.
Besonders bevorzugt werden Systeme eingesetzt, deren Dialkylaminomethylgruppen in ortho-Stellung zu aromatischen Hydroxylgruppen positioniert sind, wobei es sich bei den Alkylgruppen um gleiche oder unterschiedliche C1 bis C3-Alkylreste handelt.

Die Synthese geeigneter Mannich-Basen ist beispielsweise in DE 25 51 634 A1 und WO 2005 70984 A1 beschrieben. Bevorzugt einzusetzende Mannich-Basen sind solche auf Basis von Phenol, p-Isononylphenol oder Bisphenol A, die durch Umsetzung mit Dimethylamin und Formaldehyd z.B. nach der DE-A 2 452 531 oder Synth.Commun. (1986), 16, 1401-9 erhalten werden. Insbesondere bevorzugt sind Mannich-Basen auf Basis Phenol oder Bisphenol A.

Die Katalysatoren C) werden als Reinsubstanz oder gelöst ggf. in mehreren kleinen Portionen oder kontinuierlich eingesetzt. Insgesamt werden für die Herstellung 0,1 bis 0,8 Gew.%, vorzugsweise 0,3 bis 0,6 Gew.% Katalysator verwendet.

Die erfindungsgemäße Trimerisationsreaktion wird nach bekannten Verfahren durchgeführt, wie sie beispielsweise in WO 2005 70984 A1 beschrieben sind.

Die Trimerisierung wird in Gegenwart der Lösemittel- und/oder Verdünnungsmittel-Komponente B) durchgeführt. Die Trimerisierungsreaktion erfolgt im Temperaturbereich von 40 bis 120°C, vorzugsweise 50 bis 70°C. Die Reaktionsdauer liegt im Allgemeinen zwischen 5 und 48 Stunden, vorzugsweise 10 und 24 Stunden. Wenn der Gehalt an freiem TDI in der Reaktionsmischung unter 0,5 Gew.-% liegt, wird die Trimerisierung durch thermische Zersetzung des Katalysators oder aber bevorzugt durch Zugabe eines Katalysatorgiftes abgebrochen. Als Katalysatorgifte kommen Protonensäuren wie Dibutylphoshat oder Acylierungs- und Alkylierungsmittel wie Isophtalylsäuredichlorid bzw. Toluolsulfonsäuremethylester in Frage.

Überraschenderweise wird beim erfindungsgemäßen Verfahren mit 0,1 bis 0,8 Gew.% Ka talysator weniger Katalysator verbraucht, als bei Verwendung von herkömmlichem TDI, so dass farbhellere und vergilbungsstabilere Polyisocyanate erhalten werden.

Weiterhin kann nach beendeter Trimerisationsreaktion noch eine weitere Modifizierung des Reaktionsprodukts mit niedermolekularen oder/und polymeren Hydroxylgruppen enthaltenden Verbindungen erfolgen.

Die durch das erfindungsgemäße Verfahren hergestellten Polyisocyanate werden vorzugsweise zur Herstellung von unter dem Einfluss von Luftfeuchtigkeit aushärtbaren Beschichtungsmaterialien verwendet. Sie können ebenfalls in zur Herstellung von Haftvermittlern, Klebstoffen, Druckfarben, Dichtstoffen und Polyurethanformkörpern Verwendung finden. Besonders bevorzugt werden sie als Vernetzer in 2-Komponentensystemen mit an sich bekannten isocyanatreaktiven Verbindungen eingesetzt. Hierzu zählen beispielsweise hydroxyfunktionelle Polyether, -ester, -amide, -carbonate, -acrylate, -butadiene bzw. Mischtypen der genannten hydroxyfunktionellen Polymeren. Auch niedermolekulare Di- und Polyole, Di- und Trimerfettalkohole sowie aminofunktionelle Verbindungen können in 2K-Systemen Verwendung finden. Mit blockierten isocyanatreaktiven Verbindungen können auch Einkomponentensysteme formuliert werden, ebenso können die nach dem erfindungsgemäßen Verfahren hergestellten Produkte auch in blockierter Form als oder in Beschichtungsmaterialien eingesetzt werden. Hierbei erfolgt die Trocknung bei höheren Temperaturen bis ca. 200°C.

Neben den erfindungsgemäßen Verfahrensprodukten können in den Beschichtungen auch andere Hilfs- und Zusatzmittel wie beispielsweise die üblichen Benetzungsmittel, Verlaufsmittel, Hautverhinderungsmittel, Antischaummittel, Lösungsmittel, Mattierungsmittel wie beispielsweise Kieselsäure, Aluminiumsilikate und hochsiedende Wachse, viskositätsregulierende Stoffe, Pigmente, Farbstoffe, UV-Absorber, Stabilisatoren gegen thermischen bzw. oxidativen Abbau eingesetzt werden.

Die erhaltenen Beschichtungsmaterialien können zur Beschichtung beliebiger Substrate wie beispielsweise Holz, Kunststoffe, Leder, Papier, Textilien, Glas, Keramik, Putz, Mauerwerk, Metalle oder Beton verwendet werden. Sie lassen sich mit üblichen Applikationsmethoden wie Spritzen, Streichen, Fluten, Gießen, Tauchen, Walzen aufbringen. Die Beschichtungsmittel können in Form von Klarlacken als auch in Form pigmentierter Lacke verwendet werden.

Die aus den erfindungsgemäßen Produkten hergestellten Beschichtungen härten bei 20°C im Allgemeinen während eines Zeitraums von einigen Minuten bis Stunden zu hochwertigen Überzügen aus. Die Härtung kann jedoch auch bei tieferen Temperaturen (bis -5°C) oder beschleunigt bei höheren Temperaturen bis 200°C erfolgen.

### Beispiele

In den nachfolgenden Beispielen beziehen sich alle Prozentangaben auf das Gewicht. Zur Charakterisierung der erhaltenen Produkte wurden folgende Methoden verwendet:
Die Ermittlung des NCO-Gehaltes der in den Beispielen und Vergleichsbeispielen beschriebenen Harze erfolgte durch Titration gemäß DIN EN ISO 11 909.
Die dynamischen Viskositäten wurden nach DIN 3219 mit einem DIN Messkörper 125 bei 23 °C mit dem Viskosimeter Reolab QC der Fa. Anton Paar im Schergeschwindigkeitsbereich von 1 bis 1600 1/s gemessen.
Die Bestimmung der Restmonomergehalte erfolgte gaschromatographisch gemäß DIN EN ISO 10283.
Der Festtkörpergehalt (nicht verdampfbarer Anteil) wurde nach DIN 3251 unter den dort für Isocyanate beschriebenen Prüfbedingungen durchgeführt.
Die Farbzahlen wurden auf Basis der DIN EN 1557 mit einem LICO 400 der Firma HACH Lange in 50 mm Einweg-Rechteckküvetten bei 23°C gemessen.

### Beispiel 1 (erfindungsgemäß)

In einem mit Stickstoff gespülten 1000ml Doppelmantelplanschliffgefäß werden 426,0 g eines technischen Gemisches von 2,4- und 2,6- Toluylendiisocyanat im Verhältnis 4:1 und einem Gehalt an CIMCH von 4,8 Gew.-ppm zusammen mit 436,5 g Butylacetat vorgelegt. Das Reaktionsgemisch wird auf die gewünschte Reaktionstemperatur 75°C erhitzt. Die Trimerisationsreaktion wird durch kontinuierliche Zugabe von 7,5 g/h einer 30 Gew.-% Aktivator Lösung (Xylol) einer Mannich-Base auf Basis Bisphenol A / Fomalin / Dimethylamin gestartet. Nachdem die Trimerisationsreaktion merklich angesprungen ist, wird die kontinuierliche Aktivator Dosierung unterbrochen um die freiwerdende Reaktionsenergie sicher abzuführen. Mit Wiedererreichen der gewünschten Reaktionstemperatur wird die Aktivator Dosierung fortgesetzt. Nach ca. 7,5 Stunden ist die gesamte Menge an Aktivator Lösung 21g zudosiert und es wird unter Einhaltung der bisherigen Reaktionstemperatur bis zum Erreichen des gewünschten NCO-Gehaltes für ca. 12 Stunden nachgerührt. Zur sicheren Beendigung der Trimerisationsreaktion wird das Reaktionsgemisch mit dem molar 1,15-fachen an Toluolsulfonsäuremethylester (TSE) versetzt. Nach der Zugabe von 12,2g TSE wird das Reaktionsprodukt für eine Stunde bei 80°C nachgerührt. Das auf diese Weise erhaltende Lösungsmittel und Isocyanauratgruppen haltige Polyisocyanat weist folgende Kennzahlen auf:
NCO-Gehalt = 7,97 Gew.-%
Viskosität = 1290mPa*s @23°C
Restmonomergehalt = 0,14 Gew.-%
Festkörperanteil = 51,2 Gew.-%
APHA-Farbzahl = 73 Hazen

### Beispiel 2 (erfindungsgemäß)

Man verfährt analog zu Beispiel, 1 führt die Reaktion jedoch mit Toluylendiisocyanat mit einem 2,4-Anteil von rund 80 Gew.-% durch, welches durch Gasphasenphosgenierung hergestellt wurde und einen Gehalt an CIMCH unterhalb der Nachweisgrenze (< 1 Gew.-ppm) aufweist. Für das Erreichen des gewünschten NCO-Gehaltes werden lediglich 15,3g der identischen 30 Gew.-% Aktivator Lösung benötigt. Für die Desaktivierung wird eine Menge an TSE von 7,6g verwendet. Das so erhaltene Lösungsmittel und Isocyanauratgruppen haltige Polyisocyanat weist folgende Kennzahlen auf:
NCO-Gehalt = 8,06 Gew.-%
Viskosität = 1240mPa*s @23°C
Restmonomergehalt = 0,07 Gew.-%
Festkörperanteil = 51,1 Gew.-%
APHA-Farbzahl = 53 Hazen

### Beispiel 3 (nicht erfindungsgemäß)

Bei der Verwendung einer Toluylendiisocyanat Qualität mit einem 2,4-Anteil von rund 80 Gew.-% welche einen Gehalt an CIMCH von 160 Gew.-ppm verfährt man analog zu Beispiel 1, benötigt jedoch für den gewünschten Reaktionsumsatz 33,6g an in Beispiel 1 genannte 30 Gew.-% Aktivator Lösung, womit die Deaktivierung durch Zugabe von 19,6g Toluolsulfonsäuremethylester erfolgt. Das so erhaltende Lösungsmittel und Isocyanauratgruppen haltige Polyisocyanat weist folgende Kennzahlen auf:
NCO-Gehalt = 8,03 Gew.-%
Viskosität = 1150mPa*s @23°C
Restmonomergehalt = 0,17 Gew.%
Festkörperanteil = 51,3 Gew.-%
APHA-Farbzahl = 140 Hazen

### Beispiel 4 (nicht erfindungsgemäß)

Man verfährt analog zu Beispiel 1, führt die Reaktion jedoch mit Toluylendiisocyanat mit einem 2,4-Anteil von rund 80 Gew.-% durch, welches einen Gehalt an CIMCH von 60 Gew.-ppm aufweist. Hierbei wird für das Erreichen des gewünschten NCO-Gehaltes eine Menge von 28,5g an 30 Gew.-% Aktivator Lösung benötigt. Die Reaktionsmischung wird durch Zugabe von 16,6g Toluolsulfonsäuremethylester desaktiviert. Das so erhaltene Lösungsmittel und Isocyanuratgruppen haltige Polyisocyanat weist folgende Kennzahlen auf:
NCO-Gehalt = 8,03 Gew.-%
Viskosität = 1300mPa*s @23°C
Restmonomergehalt = 0,20 Gew.-%
Festkörperanteil = 51,0 Gew.-%
APHA-Farbzahl = 125 Hazen

### Anwendungsbeispiel Vergilbung einer Lackformulierung

Die Polyisocyanate aus Beispiel 1 und Beispiel 4 werden jeweils mit einem handelsüblichen Polyesterpolyol (®Desmophen 1300, Handelsprodukt der Bayer AG, Hydroxyl-Gehalt 4 Gew.-%) zu einer Gesamtkonzentration von 40 Gew.-% in Butylacetat im NCO/OH-Verhältnis 0,7:1 abgemischt. Die so hergestellten Formulierungen werden auf einer standardisierten weißen Platte mit einer Schichtdicke von 180µm nass aufgezogen. Die so aufgetragenen Beschichtungen werden 7 Tage bei Zimmertemperatur getrocknet, was als Vergilbungszeitpunkt t = 0 definiert wird. Nach erfolgter Bestimmung des 0-Wertes werden die Platten ungeschützt der natürlichen Bewitterung ausgesetzt und nach vorher festgelegten Zeitintervallen (siehe Tabelle 1) erneut vermessen. Alle Messungen wurden mit Hilfe des Farbmessgerätes color-guide *45*°/*0*°, (Firma BYK-Gardner) durchgeführt. Die Farbentwicklungen sind in der Tabelle 1 zusammengefasst, der Gesamtfarbabstand wird in der dem Fachmann bekannten Weise mit ΔE* ausgedrückt.

| Muster | ΔE* 7 Tage | ΔE* 14 Tage | ΔE* 1 Monat | ΔE* 3 Monate | ΔE* 6 Monate |
|---|---|---|---|---|---|
| Polyisocyanat aus Beispiel 1 CIMCH 5×1Q⁻⁴ Gew.% | 1,51 | 2,42 | 5,36 | 9,42 | 13,66 |
| Polyisocyanat aus Beispiel 2 CIMCH <10⁻⁵ Gew.% | 1,51 | 2,41 | 5,30 | 8,71 | 12,42 |
| Polyisocyanat aus Beispiel 3 CIMCH 1,6×10⁻² Gew.% | 1,62 | 2,85 | 6,92 | 15,11 | 15,02 |
| Polyisocyanat aus Beispiel 4 CIMCH 6×10-3 Gew.% | 1,58 | 2,76 | 6,58 | 12,86 | 24,78 |

Die Anwendungsbeispiele zeigen, dass die mit den erfindungsgemäßen Polyisocyanaten aus Beispiel 1 und 2 hergestellten Lackformulierungen deutlich farbstabiler sind.

## Patentansprüche

1. Verfahren zur Herstellung von Lösemittel und/oder Verdünnungsmittel , sowie Isocyanuratgruppen enthaltender Polyisocyanate auf Basis von 2,4- und/oder 2,6- Toluylendiisocyanat mit einem Gehalt an monomerem Diisocyanat von < 0,5 Gew.-% bezogen auf Polyisocyanat plus Lösemittel, durch Trimerisierung von
A) 20 bis 80 Gew.-% großtechnisch verfügbarer Gemische bestehend im Wesentlichen aus 2,4- Toluylendiisocyanat und 2,6- Toluylendiisocyanat enthaltend 65 bis 95 Gew.-% 2,4- Toluylendiisocyanat und 5 bis 35 Gew.-% 2,6- Toluylendiisocyanat in Gegenwart von
B) 20 bis 80 Gew.-% Lösemitteln und/oder Verdünnungsmitteln sowie
C) Dialkylaminomethylgruppen enthaltenden phenolischen Katalysatoren
bei einer Temperatur von 40 bis 120°C bis zum nahezu vollständigen Umsatz und anschließende Deaktivierung des Katalysators durch Zusatz von sauer reagierenden Substanzen oder durch Umsetzung mit Alkylierungsmitteln, **dadurch gekennzeichnet,**
**dass** das eingesetzte Toluylendiisocyanat einen Gehalt an 2-Chloro-6-isocyanatomethylcyclohexadienen (CIMCH) von <5 Gew.-ppm aufweist.

2. Verfahren nach Anspruch 1, **dadurch gekennzeichnet, dass** das eingesetzte Toluylendiisocyanat durch Gasphasenphosgenierung hergestellt wurde.

3. Verfahren nach Anspruch 1 und 2, **dadurch gekennzeichnet, dass** das eingesetzte Toluylendiisocyanat einen Gehalt an 2-Chloro-6-isocyanato-methylcyclohexadienen unterhalb der Nachweisgrenze von 1 Gew.-ppm aufweist.

4. Verfahren nach Anspruch 1 bis 3, **dadurch gekennzeichnet, dass** die Lösemittel und/oder Verdünnungsmittel, sowie Isocyanuratgruppen aufweisenden Polyisocyanate APHA-Farbzahlen <100 Hazen, bevorzugt <75 Hazen, besonders bevorzugt <55 Hazen aufweisen.

5. Verfahren nach Anspruch 1 bis 4, **dadurch gekennzeichnet, dass** man als Toluylendiisocyanat 2,4-Toluylendiisocyanat oder dessen technische Gemische mit bis zu 35 Gew.-% bezogen auf Gemisch an 2,6-Toluylendiisocyanat verwendet.

6. Verfahren nach Anspruch 1 bis 5, **dadurch gekennzeichnet, dass** man Katalysatoren einsetzt, die als Mannichbasen auf Basis von Phenol oder Bisphenol A durch Umsetzung mit Dimethylamin und Formaldehyd erhalten werden.

7. Verfahren nach Anspruch 1 bis 6, **dadurch gekennzeichnet, dass** man als Katalysatorgift zur Abstoppung Dibutylphosphat oder p-Tolulsäuremethylester einsetzt.

8. Lösemittel und/oder Verdünnungsmittel sowie Isocyanuratgruppen enthaltenden Polyisocyanate, erhältlich nach einem Verfahren gemäß einem der Ansprüche 1 bis 7.

9. Verwendung der Isocyanuratgruppen enthaltenden Polyisocyanate gemäß Anspruch 8 als Polyisocyanatkomponente in Polyurethanlacken.

10. Verwendung gemäß Anspruch 9 als Vernetzer in Zweikomponenten-Polyurethanlacken.

11. Verwendung der Isocyanuratgruppen enthaltenden Polyisocyanate gemäß Anspruch 8 als Polyisocyanatkomponente in Polyurethanklebstoffen.

## Claims

1. Method for producing polyisocyanates comprising solvent and/or diluent and isocyanurate groups, based on 2,4- and/or 2,6-tolylene diisocyanate, having a content of monomeric diisocyanate of < 0.5 wt.%, based on polyisocyanate plus solvent, by trimerising
A) from 20 to 80 wt.% of industrially available mixtures comprising substantially 2,4-tolylene diisocyanate and 2,6-tolylene diisocyanate comprising from 65 to 95 wt.% 2,4-tolylene diisocyanate and from 5 to 35 wt.% 2,6-tolylene diisocyanate in the presence of
B) from 20 to 80 wt.% of solvents and/or diluents and
C) phenolic catalysts comprising dialkylaminomethyl groups
at a temperature of from 40 to 120°C to almost complete conversion, and then deactivating the catalyst by addition of acid-reacting substances or by reaction with alkylating agents, **characterised in that**
the tolylene diisocyanate used has a content of 2-chloro-6-isocyanato-methylcyclohexadienes (CIMCH) of < 5 wt.ppm.

2. Method according to claim 1, **characterised in that** the tolylene diisocyanate used has been produced by gas phase phosgenation.

3. Method according to claim 1 and 2, **characterised in that** the tolylene diisocyanate used has a content of 2-chloro-6-isocyanato-methylcyclohexadienes below the detection limit of 1 wt.ppm.

4. Method according to claim 1 to 3, **characterised in that** the polyisocyanates comprising solvent and/or diluent and isocyanurate groups have APHA colour indices < 100 Hazen, preferably < 75 Hazen, particularly preferably < 55 Hazen.

5. Method according to claim 1 to 4, **characterised in that** there is used as the tolylene diisocyanate 2,4-tolylene diisocyanate or commercial mixtures thereof with up to 35 wt.%, based on the mixture, of 2,6-tolylene diisocyanate.

6. Method according to claim 1 to 5, **characterised in that** catalysts are used which are obtained as Mannich bases based on phenol or bisphenol A by reaction with dimethylamine and formaldehyde.

7. Method according to claim 1 to 6, **characterised in that** dibutyl phosphate or p-toluic acid methyl ester is used as catalyst poison for stopping the reaction.

8. Polyisocyanates comprising solvent and/or diluent and isocyanurate groups, obtainable by a method according to any one of claims 1 to 7.

9. Use of the polyisocyanates comprising isocyanurate groups according to claim 8 as the polyisocyanate component in polyurethane coatings.

10. Use according to claim 9 as the crosslinker in two-component polyurethane coatings.

11. Use of the polyisocyanates comprising isocyanurate groups according to claim 8 as the polyisocyanate component in polyurethane adhesives.

## Revendications

1. Procédé pour la préparation de polyisocyanates, contenant des solvants et/ou des diluants ainsi que des groupes isocyanurate, à base de diisocyanate de 2,4-toluylène et/ou de 2,6-toluylène présentant une teneur en diisocyanate monomère < 0,5% en poids par rapport au polyisocyanate plus solvants, par trimérisation de
A) 20 à 80% en poids de mélanges disponibles à l'échelle industrielle, essentiellement constitués par du diisocyanate de 2,4-toluylène et du diisocyanate de 2,6-toluylène contenant 65 à 95% en poids de diisocyanate de 2,4-toluylène et 5 à 35% en poids de diisocyanate de 2,6-toluylène en présence de
B) 20 à 80% en poids de solvants et/ou de diluants ainsi que de
C) catalyseurs phénoliques contenant des groupes dialkylaminométhyle
à une température de 40 à 120°C jusqu'à la conversion quasiment complète et par désactivation consécutive du catalyseur par addition de substances à réaction acide ou par réaction avec des agents d'alkylation,
**caractérisé en ce que** le diisocyanate de toluylène utilisé présente une teneur en 2-chloro-6-isocyanatométhylcyclohexadiènes (CIMCH) < 5 ppm en poids.

2. Procédé selon la revendication 1, **caractérisé en ce que** le diisocyanate de toluylène a été préparé par phosgénation en phase gazeuse.

3. Procédé selon la revendication 1 et 2, **caractérisé en ce que** le diisocyanate de toluylène utilisé présente une teneur en 2-chloro-6-isocyanatométhylcyclohexadiènes inférieure à la limite de détection de 1 ppm en poids.

4. Procédé selon la revendication 1 à 3, **caractérisé en ce que** les polyisocyanates présentant des solvants et/ou des diluants ainsi que des groupes isocyanurate présentent des indices de couleur APHA < 100 sur l'échelle de Hazen, de préférence < 75 sur l'échelle de Hazen, de manière particulièrement préférée < 55 sur l'échelle de Hazen.

5. Procédé selon la revendication 1 à 4, **caractérisé en ce qu'**on utilise, comme diisocyanate de toluylène, du diisocyanate de 2,4-toluylène ou ses mélanges techniques avec jusqu'à 35% en poids, par rapport au mélange, de diisocyanate de 2,6-toluylène.

6. Procédé selon la revendication 1 à 5, **caractérisé en ce qu'**on utilise des catalyseurs, qui sont obtenus en tant que bases de Mannich à base de phénol ou de bisphénol A par réaction avec de la diméthylamine et du formaldéhyde.

7. Procédé selon la revendication 1 à 6, **caractérisé en ce qu'**on utilise, comme poison pour le catalyseur pour l'arrêt, du phosphate de dibutyle ou de l'ester méthylique de l'acide p-toluique.

8. Polyisocyanates contenant des solvants et/ou des diluants ainsi que des groupes isocyanurate, pouvant être obtenus selon un procédé selon l'une quelconque des revendications 1 à 7.

9. Utilisation des polyisocyanates contenant des groupes isocyanurate selon la revendication 8 comme composant de type polyisocyanate dans des laques à base de polyuréthane.

10. Utilisation selon la revendication 9 comme réticulant dans des laques à base de polyuréthane à deux composants.

11. Utilisation des polyisocyanates contenant des groupes isocyanurate selon la revendication 8 comme composant de type polyisocyanate dans des adhésifs à base de polyuréthane.
